# EUROPEAN PATENT SPECIFICATION

(11) **EP 1 323 390 B1**
(45) Date of publication and mention of the grant of the patent: **21.09.2011**
(21) Application number: 01130475.5
(22) Date of filing: 20.12.2001
(51) Int. Cl.: A61B 17/70

(54) **Spinal fixation and retrieval device**
System zur Fixierung der Wirbelsäule
Système de fixation vertèbral

(43) Date of publication of application: 02.07.2003
(73) Proprietor: A-Spine Asia Co., Ltd., Taipei (TW)
(72) Inventor: Lin, Chih-I, Chino Hills, CA 91709 (US)
(74) Representative: Grünecker, Kinkeldey, Stockmair & Schwanhäusser Anwaltssozietät

(56) References cited:
- WO-A-00/59388
- WO-A-95/02373
- WO-A-96/09012
- US-A- 3 744 488
- US-A1- 2001 047 172

## Description

### FIELD OF THE INVENTION

The present invention relates generally to a spinal fixation and retrieval device, and more particularly to a spinal fixation and retrieval device capable of providing micromotion.

### BACKGROUND OF THE INVENTION

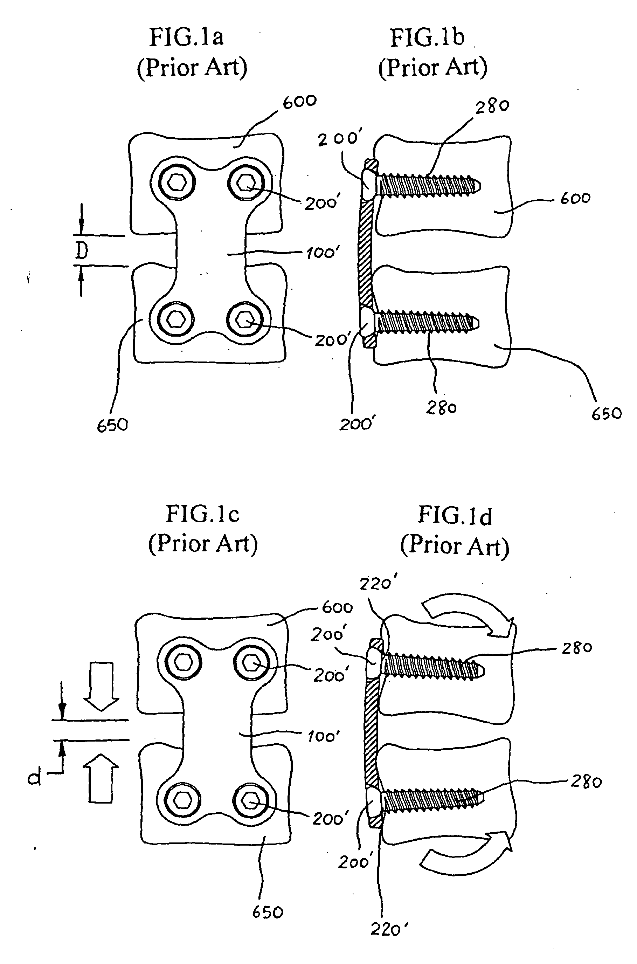

The conventional spinal fixation and retrieval device contains a rigid bone plate and bone nails, which is implanted in the body of a patient under spinal treatment with the bone nails and the bone plate being intimately joined together. In light of the intervertebral distance being caused to shorten by the body weight of the patient in the wake of surgery, the bone plate and bone nails are susceptible to falling apart. The bone nails are even vulnerable to severance. In addition, all the bone nails fastened onto the bone plate are prone to be affected, when one bone nail is out of order. As shown in FIGs. 1a, and 1b, the prior art device comprises a bone plate 100' and a plurality of screws 200'. FIG. 1a shows a schematic view taken immediately after the surgery. FIG. 1b is a side view of FIG. 1a. The prior art device is implanted into the spinal segments 600 and 650. The letter "D" denotes the distance between the two spinal segments 600 and 650. Immediately after the surgery, the bone nails 200', the spinal segments 600 and 650, and the bone plate 100' are intimately joined together such that the threaded stems 280 are parallel to each other. FIGs. 1c and 1d are schematic views taken in a period after the surgery. As shown in FIG. 1c, the distance "d" becomes smaller, due to the body weight of the patient. As shown in FIG. 1d, the threaded stems 280 are no longer parallel to each other; they form therebetween an acute angle. As a result, the bone nails 200' are apt to become unfastened. A crack or severance may occur in the necks 220' of the bone nails 200'.

With a view to providing a solution to the problem described above, this inventor of the present invention disclosed a drawer-type vertebral auxiliary fixation device capable of providing micromotion in US patent No. 5,616,142. However, this drawer-type device is by no means free of deficiency. For example, the drawer-type device can not be easily bent to conform to the spinal curvature. In addition, the structural strength of the drawer-type device is often inadequate unless the device has an appropriate height. Moreover, the drawer-type device does not have a supporting force at the time when the surgery is completed.

The international application WO 00/59388 A discloses a spiral device as defined in the preamble of claim 1. A ligament body is fixed with bone fasteners being screwed to vertebrae. Fasteners are permitted limited relative motion within the openings. It is taught that this flexibility also reduces the likelihood of the fasteners backing out over time.

The US patent application US 2001/0047172 A1 presents a bone fixation system, wherein a plate is engaged to the spine. The plate comprises holes and slots that enable the insertion of bone screws, which are allowed to translate within the slots while an additional retainer assembly prevents screw backout. The angular adjustment of the bone screw is prevented by the snug fit of the screw in the bore.

WO 96/09012 A discloses a fixation plate for long bone fractures; thus, not meant for the stabilization of vertebrae. The plate comprises a slot and holes. Screws can be engaged with the bone portion via the sloped holes for the compression of the bone with a compression device, but such screws are removed once the portions of the bone are fixed by screws engaged with the bone via the slot, thereby allowing dynamic compression of the bone, as the screws are able to slide in the slots, and thus allowing improved healing of bone fractures.

WO 95/02373 A discloses an anchor inserted in a bone, wherein the anchor comprises a hollow shank for receiving screws to fix a plate to the bone. The openings of the plate, wherein the screws are inserted, are elongated slots to provide adjustment of the element along the major axis of the slot. The screws are less lively to loosen as they engage in the anchor and not in the bone.

None of the above referenced documents discloses a way to prevent screw backout without introducing other drawbacks, for example additional parts such as anchors or retainer assemblies.

It is therefore the primary objective of the present invention to provide a spinal fixation and retrieval device which is free of the drawbacks of the prior art devices described above.

In keeping with the principle of the present invention, the foregoing objective of the present invention is attained by the spinal fixation and retrieval device according to claim 1.

Preferred embodiments are defined in the dependent claims.

### BRIEF DESCRIPTION OF THE DRAWINGS

FIGs. 1 a and 1 b show a top view and a cross-sectional view of the prior art spinal fixation and retrieval device at the time when the surgery is completed.
FIGs. 1c and 1d show a top view and a cross-sectional view of the prior art spinal fixation and retrieval device after the surgery.
FIGs. 2a and 2b show a top view and a cross-sectional view of the spinal fixation and retrieval device according to a first embodiment of the present invention at the time when the surgery is completed.
FIGs. 2c and 2d show a top view and a cross-sectional view of the spinal fixation and retrieval device of the first embodiment of the present invention after the surgery is completed.
FIG. 3 shows a schematic perspective view of the spinal fixation and retrieval device of the first embodiment of the present invention.
FIG. 4a and 4b are partial lateral cross-sectional view of the spinal fixation and retrieval device of the first embodiment of the present invention.
FIG. 5 is a partial longitudinal cross-sectional view of the spinal fixation and retrieval device of the first embodiment of the present invention.
FIG. 6 show a top view of the spinal fixation and retrieval device according to a second embodiment of the present invention.

### DETAILED DESCRIPTION OF THE EMBODIMENTS

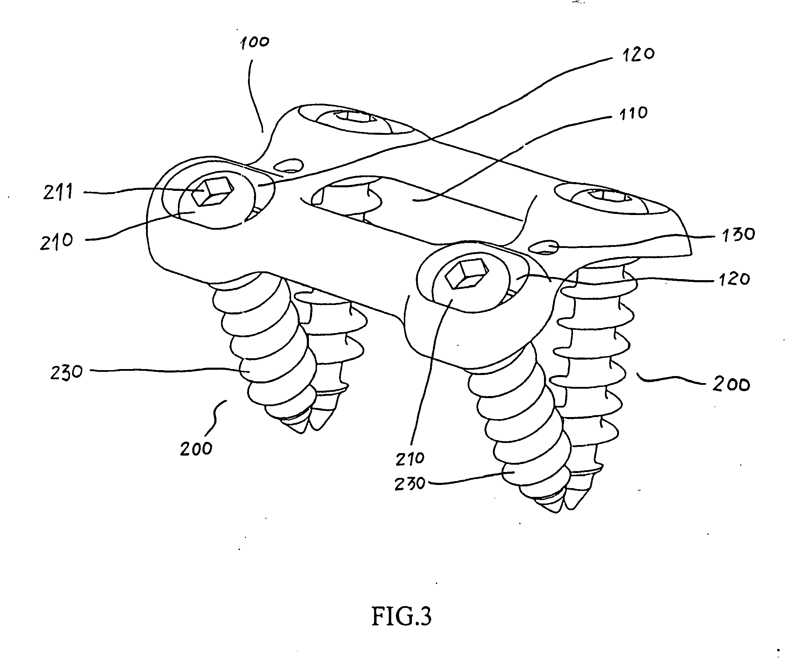

As shown in FIGs. 3 to 5, the spinal fixation and retrieval device constructed according to a first embodiment of the present invention comprises a bone plate 100 and a plurality of screws 200.

The bone plate 100 is provided with a plurality of slot holes 120, via which the screws 200 are fastened onto spinal segments under treatment. The screws have a head 210, a neck 220, and a threaded stem 230.

The slot holes 120 are long slot holes, each of which is provided at the bottom with a protruded edge 121. The head 210 of the screw has a diameter ranging between the short diameter of the long slot hole 120 and the short diameter of the protruded edge 121, and the neck 220 has a diameter is slightly smaller than the short diameter of the protruded edge 121. The diameter of the head 210 of the screw is smaller than the long diameter of the long slot hole 120. Therefore, the screws 200 will be restricted in the lateral direction of the long slot hole 120 after the plate 100 and the screws 200 are implanted onto the vertebrae, but the screws 200 and the plate 100 are able to move relatively in the longitudinal direction of the long slot hole 120.

The bone plate 100 of the present invention is similar in shape and structure to the prior art bone plates; however, at least one of the holes of the bone plate of the present invention is a long slot hole 120 having a long diameter and a short diameter. Instead of the protruded edge 121 provided at the bottom of the long slot hole 120, the long slot hole 120 may be provided with slanted outward from the bottom to the upper portion of the bone plate. In this case, the long diameter and the short diameter of the upper portion of the long slot hole are respectively greater than the long diameter and the short diameter of the bottom of the long slot hole. The word "upper" refers to a face away from the post-surgery spinal segment. The word "bottom" refers to a face toward the post-surgery spinal segment. The definitions of "upper" and "bottom" are applicable throughout the entire text of this specification.

The holes of the bone plate may be provided in a manner so that only one hole is used per spinal segment. According to the present invention, at least one of the holes is a long slot hole 120. In the event that the n number (n>2) of the spinal segments are to be fixed and only one hole is used per spinal segment, preferably n-1 number of the holes of the bone plate are long slot holes 120. If the holes of the bone plate are provided in a manner so that a pair of holes are used per spinal segment, at least one pair of the holes are long slot holes 120 according to the present invention. In the same pattern, for the bone plate fixing n (n>2) spinal segments and only one pair of holes are used per spinal segment, preferably n-1 pairs of the holes of the bone plate are long slot holes 120. Of course, all slot holes may be long slot holes as shown in the first embodiment of the present invention. The bone plate may be further provided with a center hole 110 and locating holes 130, which are located between the slot holes 120, as shown in FIG. 3. The bone plate may be further provided with one or more tool holes. The center hole, tool hole, and locating hole are optional.

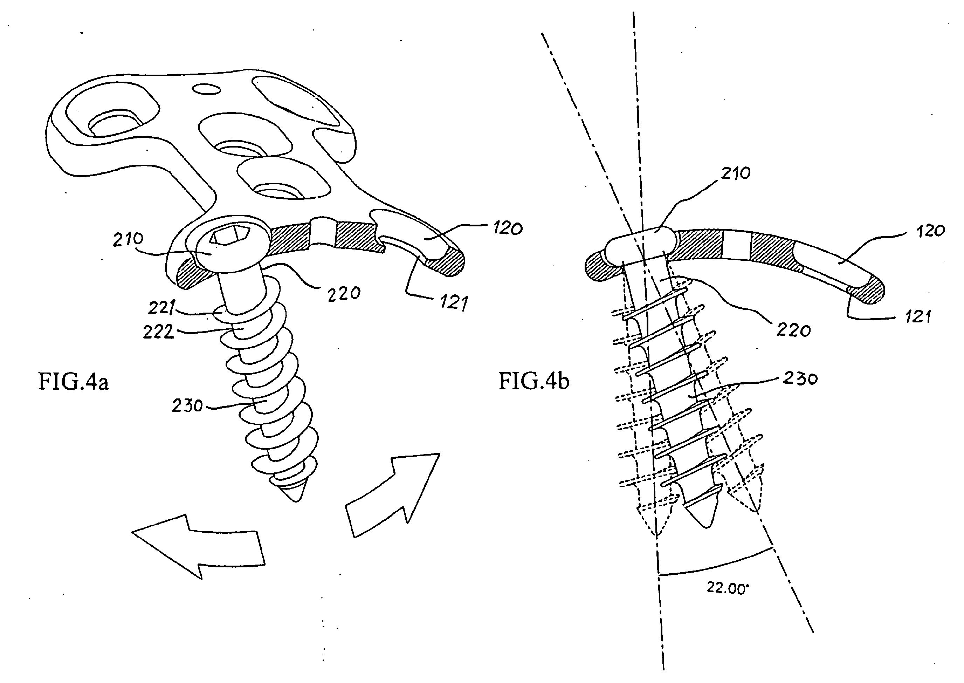

The screws used in the present invention are similar in construction to the prior art screws having a head, a neck, and a threaded stem; preferably, the threaded stem has a special thread. The head 210 is of any shape, preferably, round shape. The head 210 has a diameter which is substantially smaller than the short diameter of the upper portion of the long slot hole 120 and is greater than the short diameter of the protruded edge 121 of the long slot hole 120. The head 210 may be further provided with a tool hole 211. The neck 220 has a diameter which is slightly smaller than the short diameter of the protruded edge 121 of the long slot hole. The threaded stem 230 as shown in FIG. 4a has a first spiral ridge 221 contiguous to the neck 220 and a first shank 222. Preferably, the short diameter of the protruded edge 121 of the long slot hole ranges between the diameter of the first spiral ridge 221 and the diameter of the first shank 222, thereby enabling the screw 200 to be better equipped to join with the bone plate 100. The protruded edge 121 has a thickness, which is smaller than the pitch of the thread stem 230. Preferably, the thickness of the protruded edge 121 is substantially about equal to the pitch of the threaded stem 230. The threaded stem 230 of the screw 200 is gradually smaller in diameter from the first spiral ridge 221, and may be equal in diameter.

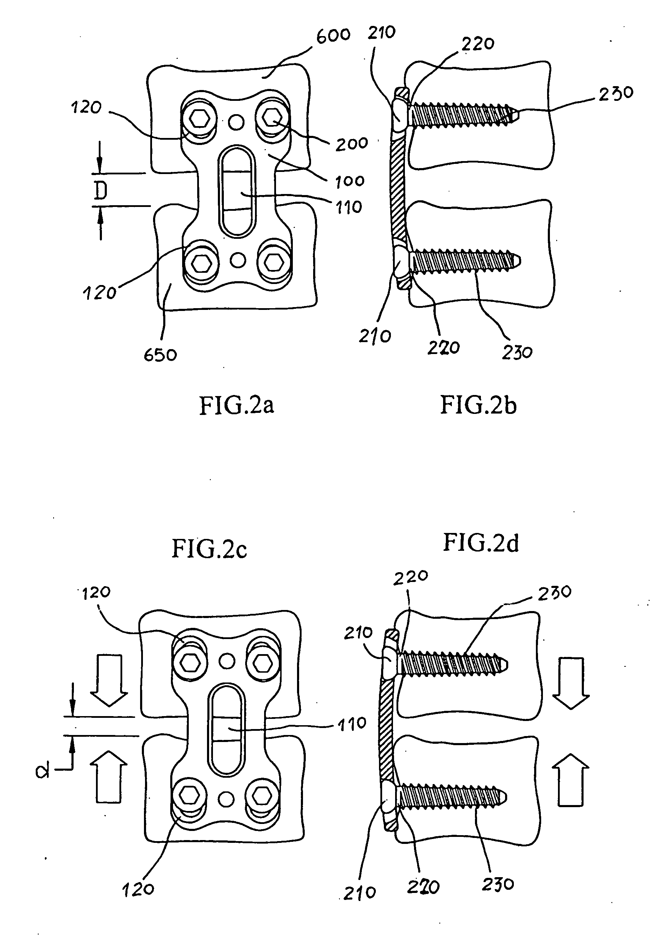

The device of the present invention is implanted into the spinal segments 600 and 650, as shown in FIGs. 2a and 2b. The letter "D" denotes the distance between the two spinal segments 600 and 650. Immediately after the surgery, the screws 200, the spinal segments 600 and 650, and the bone plate 100 are intimately joined together such that the threaded stems 230 are parallel to each other. As shown in FIGs. 2c and 2d, in a period after the surgery, the distance between the spinal segments 600 and 650 "d" becomes smaller because of the pressure of body weight or other factors. Accordingly, the screws 200 are caused to displace in the slot holes 120 and move inward. The word "inward" refers to the direction toward the center of the bone plate 100.

FIGs. 4a and 4b show that the device of the present invention can be implanted at various angles in the lateral direction, with the maximum angle being 22 degrees. The allowable angle can be much greater by a design change in the protruded edge 121 and the head 210 of the screws 200. The bone plate shown in FIGs. 4a and 4b is provided with two center holes.

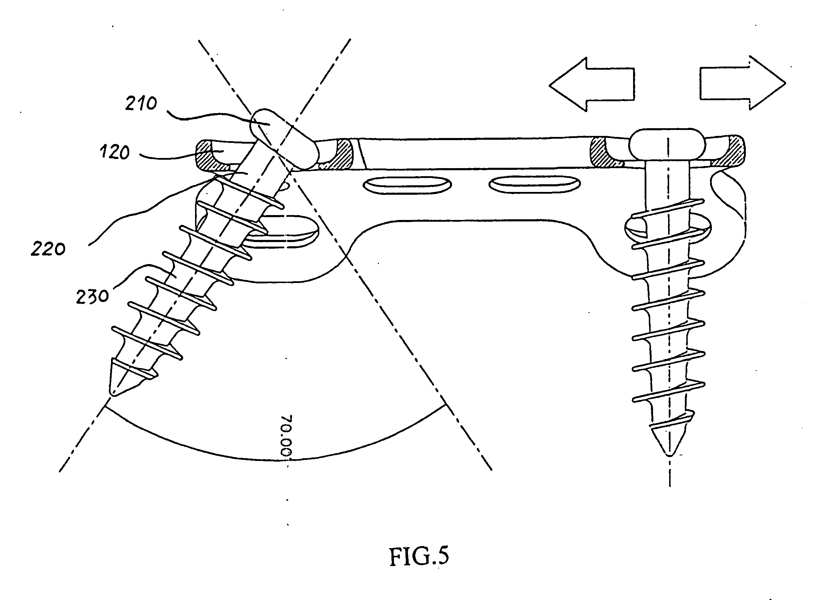

FIG. 5 is similar to FIG. 4b, but in the longitudinal direction. The screw 200 can be fastened onto the spinal segment via the long slot hole at various angles in the longitudinal direction, with the maximum angle being 70 degrees. The bone plate shown in FIG. 5 is provided with two center holes.

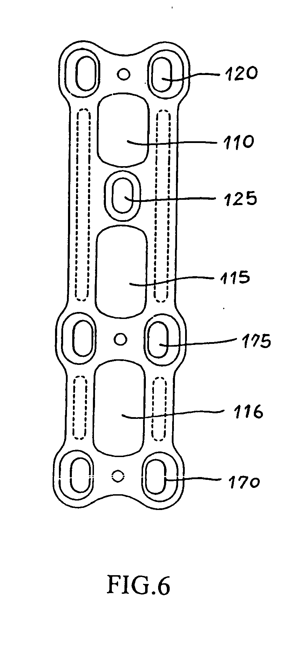

The spinal fixation and retrieval device according to a second embodiment of the present invention is shown in FIG. 6, wherein the reference numerals of 110, 115, and 116 denote the center holes, whereas the reference numerals of 120, 125, 170, and 175 denote the long slot holes. The bone plate is intended for use in fixing four spinal segments. The bone plate can be easily bent to conform to the curvatures of the four spinal segments, thanks to the center holes 110, 115, and 116.

## Claims

1. A spinal fixation and retrieval device comprising:
a bone plate (100) having a plurality of slot holes (120); and
a plurality of screws (200), said screws (200) are adapted to be fastened via said slot holes (120) onto vertebrae (600,650), at least one of which has a head (210), a threaded stem (230), and a neck (220) connecting said head (210) and said threaded stem (230);
wherein at least one of said slot holes (120) is a long slot hole (120) having two opposite walls defining a width of said long slot hole (120), and said two opposite walls further being slanted outward from a bottom portion to an upper portion thereof; wherein said head (210) has a diameter ranging between a relatively longer distance between said upper portions and a relatively shorter distance between said bottom portions of said two opposite walls, said threaded stem (230) comprises a first spiral ridge (221) adjacent to said neck (220), and a shank (222) next to said first spiral ridge (221) which has a diameter smaller than said relatively shorter distance between said bottom portions of said two opposite walls,
**characterized in that** said first spiral ridge (221) has a diameter greater than said relatively shorter distance between said bottom portions of said two opposite walls.

2. The device as defined in claim 1, wherein said neck (220) has a diameter smaller than said relatively shorter distance between said bottom portions of said two opposite walls.

3. The device as defined in claim 1, wherein said bottom portions of said two opposite walls have a thickness substantially smaller than a pitch between said first spiral ridge (221) and a second spiral ridge next to said first spiral ridge (221).

4. The device as defined in claim 1, wherein said long slot hole (120) is provided with a protruded edge (121) at a bottom of each of two opposite walls defining a width thereof; wherein said head (210) has a diameter ranging between said width of said long slot hole (120) and a distance between said protruded edges (121).

5. The device as defined in claim 4, wherein said long slot hole (120) is slanted outward from said bottom to an upper portion thereof.

6. The device as defined in claim 4, wherein said neck (220) has a diameter smaller than said distance between said protruded edges (121).

7. The device as defined in claim 4, wherein said first spiral ridge (221) has a diameter greater than said distance between said protruded edges (121) and said shank (222) has a diameter smaller than said distance between said protruded edges (121).

8. The device as defined in claim 7, wherein said protruded edges (121) have a thickness substantially smaller than a pitch between said first spiral ridge (221) and a second spiral ridge next to said first spiral ridge (221).

9. The device as defined in claim 7 or 8, wherein said first spiral ridge (221) has the greatest diameter in said threaded stem (230).

## Patentansprüche

1. Wirbelsäulenfixier- und Wiederherstellungsvorrichtung, umfassend:
eine Knochenplatte (100) mit einer Mehrzahl von Schlitzlöchern (120); und
eine Mehrzahl von Schrauben (200), wobei die Schrauben (200) dafür ausgelegt sind,
über die Schlitzlöcher (120) an Wirbeln (600, 650) befestigt zu werden, wobei wenigstens eine hiervon einen Kopf (210), einen Gewindekörper (230) und einen den Kopf (210) und den Gewindekörper (230) verbindenden Hals (220) aufweist;
wobei wenigstens eines von den Schlitzlöchern (120) ein Langschlitzloch (120) ist, das zwei gegenüberliegende Wände aufweist, die eine Breite des Langschlitzloches (120) festlegen, und die beiden gegenüberliegenden Wände des Weiteren von einem Bodenabschnitt zu einem oberen Abschnitt hiervon nach außen schräggestellt sind; wobei der Kopf (210) einen Durchmesser in einem Bereich zwischen einem vergleichsweise längeren Abstand zwischen den oberen Abschnitten und einem vergleichsweise kürzeren Abstand zwischen den Bodenabschnitten der beiden gegenüberliegenden Wände aufweist, wobei der Gewindekörper (230) benachbart zu dem Hals (220) einen ersten Spiralsteg (221) und nahe dem ersten Spiralsteg (221) einen Schaft (222) umfasst, der einen Durchmesser aufweist, der kleiner als der vergleichsweise kürzere Abstand zwischen den Bodenabschnitten der beiden gegenüberliegenden Wände ist,
**dadurch gekennzeichnet, dass**
der erste Spiralsteg (221) einen Durchmesser aufweist, der größer als der vergleichsweise kürzere Abstand zwischen den Bodenabschnitten der beiden gegenüberliegenden Wände ist.

2. Vorrichtung nach Anspruch 1, wobei der Hals (220) einen Durchmesser aufweist, der kleiner als der vergleichsweise kürzere Abstand zwischen den Bodenabschnitten der beiden gegenüberliegenden Wände ist.

3. Vorrichtung nach Anspruch 1, wobei die Bodenabschnitte der beiden gegenüberliegenden Wände eine Dicke aufweisen, die im Wesentlichen kleiner als eine Ganghöhe zwischen dem ersten Spiralsteg (221) und einem zweiten Spiralsteg nahe dem ersten Spiralsteg (221) ist.

4. Vorrichtung nach Anspruch 1, wobei das Langschlitzloch (120) mit einer vorstehenden Kante (121) an einem Boden einer jeden der beiden gegenüberliegenden Wände zur Festlegung einer Breite hiervon versehen ist; wobei der Kopf (210) einen Durchmesser in einem Bereich zwischen der Breite des Langschlitzloches (120) und einem Abstand zwischen den vorstehenden Kanten (121) aufweist.

5. Vorrichtung nach Anspruch 4, wobei das Langschlitzloch (120) vom Boden zu einem oberen Abschnitt hiervon nach außen schräggestellt ist.

6. Vorrichtung nach Anspruch 4, wobei der Hals (220) einen Durchmesser aufweist, der kleiner als der Abstand zwischen den vorstehenden Kanten (121) ist.

7. Vorrichtung nach Anspruch 4, wobei der erste Spiralsteg (221) einen Durchmesser aufweist, der größer als der Abstand zwischen den vorstehenden Kanten (121) ist, und der Schaft (222) einen Durchmesser aufweist, der kleiner als der Abstand zwischen den vorstehenden Kanten (121) ist.

8. Vorrichtung nach Anspruch 7, wobei die vorstehenden Kanten (121) eine Dicke aufweisen, die im Wesentlichen kleiner als eine Ganghöhe zwischen dem ersten Spiralsteg (221) und einem zweiten Spiralsteg nahe dem ersten Spiralsteg (221) ist.

9. Vorrichtung nach Anspruch 7 oder 8, wobei der erste Spiralsteg (221) den größten Durchmesser in dem Gewindekörper (230) aufweist.

## Revendications

1. Dispositif de fixation vertébral comprenant :
une plaque osseuse (100) comportant une pluralité de trous oblongs (120) ; et
une pluralité de vis (200), lesdites vis (200) étant adaptées pour être fixées via lesdits trous oblongs (120) sur les vertèbres (600, 650), au moins une d'entre elles possédant une tête (210), une tige filetée (230) et un col (220) reliant ladite tête (210) et ladite tige filetée (230) ;
dans lequel au moins un desdits trous oblongs (120) est un long trou oblong (120) possédant deux parois opposées définissant une largeur dudit long trou oblong (120), et lesdites deux parois opposées étant en outre inclinées vers l'extérieur depuis une partie inférieure vers une partie supérieure de celles-ci ; dans lequel ladite tête (210) présente un diamètre compris entre une distance relativement plus longue entre lesdites parties supérieures et une distance relativement plus courte entre lesdites parties inférieures desdites deux parois opposées, ladite tige filetée (230) comprend une première arête en spirale (221) adjacente audit col (220) et un fût (222) proche de ladite première arête en spirale (221) qui présente un diamètre inférieur à ladite distance relativement plus courte entre lesdites parties inférieures desdites deux parois opposées,
**caractérisé en ce que** ladite première arête en spirale (221) présente un diamètre plus grand que ladite distance relativement plus courte entre lesdites parties inférieures desdites deux parois opposées.

2. Dispositif selon la revendication 1, dans lequel ledit col (220) présente un diamètre inférieur à ladite distance relativement plus courte entre lesdites parties inférieures desdites deux parois opposées.

3. Dispositif selon la revendication 1, dans lequel lesdites parties inférieures desdites deux parois opposées présentent une épaisseur sensiblement inférieure à un pas entre ladite première arête en spirale (221) et une seconde arête en spirale proche de ladite première arête en spirale (221).

4. Dispositif selon la revendication 1, dans lequel ledit long trou oblong (120) est doté d'un rebord en saillie (121) au niveau d'un fond de chacune des deux parois opposées définissant une largeur entre elles ; dans lequel ladite tête (210) présente un diamètre compris entre ladite largeur dudit long trou allongé (120) et une distance entre lesdits rebords en saillie (121).

5. Dispositif selon la revendication 4, dans lequel ledit long trou oblong (120) est incliné vers l'extérieur depuis ledit fond vers une partie supérieure de celui-ci.

6. Dispositif selon la revendication 4, dans lequel ledit col (220) présente un diamètre inférieur à ladite distance entre lesdits rebords en saillie (121).

7. Dispositif selon la revendication 4, dans lequel ladite première arête en spirale (221) présente un diamètre supérieur à ladite distance entre lesdits rebords en saillie (121) et ledit fût (222) présente un diamètre inférieur à ladite distance entre lesdits rebords en saillie (121).

8. Dispositif selon la revendication 7, dans lequel lesdits rebords en saillie (121) présentent une épaisseur sensiblement inférieure à un pas entre ladite première arête en spirale (221) et une seconde arête en spirale proche de ladite première arête en spirale (221).

9. Dispositif selon la revendication 7 ou 8, dans lequel ladite première arête en spirale (221) présente le diamètre le plus grand dans ladite tige filetée (230).
